# EUROPEAN PATENT SPECIFICATION

(11) **EP 2 460 523 B1**
(45) Date of publication and mention of the grant of the patent: **04.01.2017**
(21) Application number: 10804385.2
(22) Date of filing: 27.07.2010
(51) Int. Cl.: A61K 31/506, A61K 9/20, A61K 47/04, A61K 47/38

(54) **TABLET**
TABLETTE
COMPRIMÉ

(30) Priority: 28.07.2009 JP 2009175695
(43) Date of publication of application: 06.06.2012
(73) Proprietor: Takeda Pharmaceutical Company Limited, Osaka-shi, Osaka 541-0045 (JP)
(72) Inventor: MURAKAWA, Yusuke, Yodogawa-ku, Osaka-shi Osaka 532-0024 (JP); OKABE, Takayuki, Osaka 532-8686 (JP)
(74) Representative: Dossmann, Gérard
(86) International application number: PCT/JP2010/062568
(87) International publication number: WO 2011/013639

(56) References cited:
- WO-A1-2008/093878
- WO-A2-2008/114800
- JP-A- 9 169 641
- JP-A- 2006 070 024
- JP-A- 2007 211 005
- JAPAN PHARMACEUTICAL EXCIPIENTS COUNCIL IYAKUHIN TENKABUTSU JITEN 2007 2007, page 97, 100, 223, 382, 383, 402, XP008151763

## Description

### Technical Field

The present invention relates to a preparation having a high content of 2-[[6-[(3R)-3-amino-1-piperidinyl]-3,4-dihydro-3-methyl-2,4-dioxo-1(2H)-pyrimidinyl]methyl]-4-fluorobenzonitrile (to be abbreviated as "compound (A)" in the present specification) or a salt thereof, a production method thereof and the like.

### [Background of the Invention]

Compound (A) is a compound represented by the following formula.

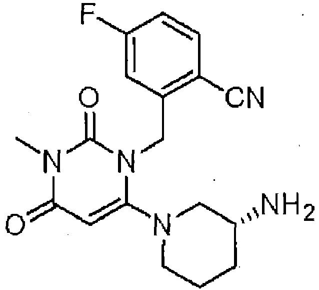

Compound (A) or a salt thereof is reported as an inhibitor of an enzyme dipeptidyl peptidase.(DPP-IV) that decomposes glucagon-like peptide-1 (GLP-1), which is a hormone enhancing insulin secretion (US2005/0261271).

In addition, a method including administering 1 - 250 mg of compound (A) or a salt thereof to a patient once a week has been reported (WO2008/033851). Compound (A) and a salt thereof are recommended to be orally administered in view of the easiness of self-administration, and a tablet, particularly a tablet in the dosage form of once per week, has been desired.

WO2008/114800 A1 discloses a tablet comprising compound A or a salt thereof. The tablet comprises a granule comprising compound A or a salt thereof and microcrystalline cellulose and a tableting aid comprising magnesium stearate and microcrystalline cellulose. The compound A is contained in a proportion of 25-40 wt% relative to the tablet. The granule can comprise additives, such as a binder (preferably hydroxypropylmethylcellulose) and a glidant. The glidant is preferably light anhydrous silicic acid, hydrated silicon dioxide.

The dosage form of once per week is expected to improve the compliance of patients; on the other hand, it requires provision of higher dose compound (A) or a salt thereof to patients as compared to a dosage form of once per day. In this case, patients may have to take tablet with a high content of compound (A). However, a higher content of compound (A) in a tablet increases the tablet size, and the compliance for patients, particularly infants and the elderly patients having difficulty in swallowing, may decrease on the contrary.

### Summary Of The Invention

### Problems to be Solved by the Invention

Accordingly, the development of a tablet with a high content of compound (A) or a salt thereof and a size permitting easy administration has been desired as a pharmaceutical product with high patient compliance.

To downsize the tablet, a tablet with a decreased content of additive was considered. As a result, a mixture containing compound (A) agglomerated during granulation, thus causing fluidity failure and granulation failure. The granulation failure decreases the yield of good products from granulation powder, causes tableting trouble in a tableting step to decrease the yield of good products of tablets, and markedly decreases tablet productivity. Therefore, the present invention aims to provide a tablet with a high content of compound (A) or a salt thereof and a size permitting easy administration, which does not cause fluidity failure and granulation failure during granulation.

### Means of Solving the Problems

The present inventors have conducted intensive studies in an attempt to solve the above-mentioned problem and found that use of a fluidizer as an additive prevents fluidity failure and granulation failure during granulation, and results in successful downsizing of a tablet with a high content of compound (A) or a salt thereof, which resulted in the completion of the present invention.

Accordingly, the present invention relates to the following:
[1] a tablet comprising compound (A) or a salt thereof, light anhydrous silicic acid, crystalline cellulose and hydroxypropylcellulose, wherein the content of compound (A) is 35 - 50 weight% (hereinafter sometimes to be referred to as "the tablet of the present invention").
[5] the tablet of the above-mentioned [1], wherein the content of crystalline cellulose is 10 - 50 weight%, and the content of hydroxypropylcellulose is 3 - 10 weight%,
[6] the tablet of any of the above-mentioned [1] or [5],
   wherein the content of compound (A) is more than 40 weight% to not more than 50 weight%,
[7] the tablet of any of the above-mentioned [1], [5] or [6], having a dosage form of once per week,
[8] a method of producing a tablet comprising 35 - 50 weight% of compound (A), comprising a step of granulating a mixture comprising compound (A) or a salt thereof and light anhydrous silicic acid, and
   a step of tableting the granulation product obtained by the aforementioned step,
   wherein the method further comprises the step of mixing, after the granulation step, the granulation product obtained by the granulation step, crystalline cellulose and hydroxypropylcellulose, wherein the granulation product obtained by the mixing step is tableted in the tableting step.
[11] a tablet obtained by the production method of the above-mentioned [8].

### Effect of the Invention

According to the present invention, a tablet with a high content of compound (A) or a salt thereof and a size permitting easy administration, which does not cause fluidity failure and granulation failure during granulation, can be provided. That is, since the tablet of the present invention is of a size permitting easy administration, it is useful as a pharmaceutical product with high patient compliance, and is particularly useful for administering a high dose of compound (A) or a salt thereof to a patient.

According to the present invention, moreover, a tablet superior in storage stability and dissolution property for a long time, and the like can be provided by combining compound (A) and a fluidizer, preferably compound (A), a fluidizer and a binder.

Furthermore, the tablet of the present invention containing crystalline cellulose and a binder other than crystalline cellulose as additives has a suitable tablet hardness, prevents breakage of tablet surface and crack of tablet that occur during the production step, as well as tableting trouble (particularly capping), and shows more superior productivity.

### [Detailed Description of the Invention]

The present invention is explained in detail in the following.

Examples of the salt of compound (A) include a pharmacologically acceptable salt, such as a salt with inorganic acid, a salt with organic acid, a salt with basic or acidic amino acid and the like.

Preferable examples of the salt with inorganic acid include salts with hydrochloric acid, hydrobromic acid, nitric acid, sulfuric acid, phosphoric acid and the like.

Preferable examples of the salt with organic acid include salts with benzoic acid, formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysine, ornithine and the like, and preferable examples of the salt with acidic amino acid include salts with aspartic acid, glutamic acid and the like.

Preferable examples of the salt of compound (A) include salts with trifluoroacetic acid, succinic acid, hydrochloric acid and the like. Of these, succinate of compound (A) is preferable, and monosuccinate of compound (A) is more preferable.

Compound (A) may be a solvent solvate (e.g., water solvate), or non-solvent solvate (e.g., non-water solvate).

Compound (A) may be labeled with an isotope (e.g., ³H, ¹⁴C, ³⁵S, ¹²⁵I).

Moreover, a deuterium conversion form wherein ¹H has been converted to ²H(D) is also encompassed in compound (A),

The metabolite of compound (A) or a salt thereof include N-demethylated compounds represented by the following formula, and the metabolite has a DPP-IV inhibitory activity.

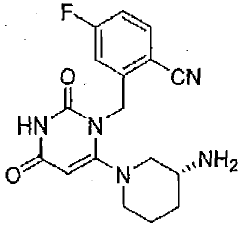

In the tablet of the present invention, compound (A) (free form) is used in an amount corresponding to a content of 35 - 50 weight%, preferably from more than 40 weight% to not more than 50 weight%, more preferably from more than 40 weight% to not more than 45 weight%, per one tablet of the present invention.

A fluidizer contained in the tablet of the present invention may be an additive (excluding crystalline cellulose) that suppresses agglomeration of a mixture containing compound (A) or a salt thereof, improves fluidity of the mixture, and prevents blocking, during the production step (particularly granulation step) of the tablet of the present invention. The fluidizer to be used in the present invention is light anhydrous silicic acid [e.g., AEROSIL 50, AEROSIL 130, AEROSIL 150, AEROSIL 200, AEROSIL 380 (trade names); Nippon Aerosil Co., Ltd. ]. Preferred is AEROSIL 200.

The fluidizer is used in an amount corresponding to a content of preferably 0.1 - 1 weight%, more preferably 0.1 - 0.6 weight%, per one tablet of the present invention.

The weight ratio of compound (A) (free form) to the fluidizer (compound (A):fluidizer) is preferably 1:0.001 - 1:0.1, more preferably 1:0.001 - 1:0.05, still more preferably 1:0.005 - 1:0.02.

The binder to be contained in the tablet of the present invention may be any additive as long as it binds particles during dry or wet granulation and direct tableting. However, light anhydrous silicic acid and hydrated silicon dioxide are excluded.

Examples of the binder to be used in the present invention include crystalline cellulose [e.g., crystalline cellulose {e.g., CEOLUS KG-802 (grade:KG-802) (trade name); CEOLUS PH-302 (grade:PH-302) (trade name); Asahi Kasei Chemicals Corporation}, crystalline cellulose (granule), crystalline cellulose (fine particle)], hydroxypropylcellulose [e.g., grades: L, SL, SSL (trade names); Nippon Soda Co., Ltd.], hydroxypropylmethylcellulose [e.g., hypromellose2910, TC-5 (grades: MW, E, EW, R, RW) (trade name); Shin-Etsu Chemical Co., Ltd.], povidone (polyvinyl pyrrolidone), copolyvidone and the like. Crystalline cellulose, hydroxypropylcellulose, hydroxypropylmethylcellulose, and povidone are preferable, and crystalline cellulose and hydroxypropylcellulose are more preferable.

The amount of the binder to be contained in one tablet of the present invention is preferably 13 - 60 weight%, more preferably 30 - 40 weight%.

For the tablet of the present invention to have a suitable tablet hardness, prevent breakage of tablet surface and crack of tablet that occur during the production step, as well as tableting trouble (particularly capping), it preferably contains 2 or more kinds of binders. In the present specification, the "tableting trouble" means unpreferable phenomena that occur during tableting, such as sticking (attachment of powder to punch), binding (large friction between die and tablet), capping (cap-like detachment of tablet), laminating (detachment of tablet into layers) and the like.

The tablet of the present invention contains two or more kinds of binders, the combination of the binders is crystalline cellulose and hydroxypropylcellulose.

As the content of each binder in one tablet of the present invention is preferably 10 - 50 weight% of crystalline cellulose, and 3 - 10 weight% of a binder other than crystalline cellulose (preferably, hydroxypropylcellulose), more preferably, 20 - 40 weight% of crystalline cellulose and 3 - 8 weight% of a binder other than crystalline cellulose (preferably, hydroxypropylcellulose).

Here, the above-mentioned crystalline cellulose is not particularly limited as long as it is used as an additive for pharmaceutical products, and one kind from crystalline cellulose having a particle size of not less than 95% (pass (passage of sieve) of 100M sieve, not less than 95%) for not more than 150 µm and bulk density of 0.13 - 0.23 g/cm³ [e.g., CEOLUS KG-802 (grade:KG-802) (trade name); Asahi Kasei Chemicals Corporation], crystalline cellulose having a particle size of 60 - 80% (pass (passage of sieve) of 100M sieve, 60 - 80%) for not more than 150 µm and bulk density of 0.35 - 0.46 g/cm³ [e.g., CEOLUS PH-302 (grade:PH-302) (trade name); Asahi Kasei Chemicals Corporation], crystalline cellulose (granule), crystalline cellulose (fine particle) and the like may be used alone or two or more kinds therefrom may be mixed and used. Preferred are crystalline cellulose (KG-802) having a particle size of not less than 95% (pass (passage of sieve) of 100M sieve, not less than 95%) for not more than 150 µm and bulk density of 0.13 - 0.23 g/cm³ and crystalline cellulose (PH-302) having a particle size of 60 - 80% (pass (passage of sieve) of 100M sieve, 60 - 80%) for not more than 150 µm and bulk density of 0.35 - 0.46 g/cm³.

Moreover, the above-mentioned hydroxypropylcellulose is not particularly limited as long as it is used as an additive for pharmaceutical products. Preferred are hydroxypropylcellulose having a viscosity in 2% water solution at 20°C of 6.0 - 10.0 mPa·s, hydroxypropylcellulose having a viscosity in 2% water solution at 20°C of 3.0 - 5.9 mPa·s, hydroxypropylcellulose having a viscosity in 2% water solution at 20°C of 2.0 - 2.9 mPa·s and the like, and more preferred is hydroxypropylcellulose (preferably, fine powder) having a viscosity in 2% water solution at 20°C of 6. 0 - 10.0 mPa·s.

In addition, the hydroxypropylcellulose fine powder having a viscosity of 6.0 - 10.0 mPa·s in 2% water solution at 20°C preferably has a particle size of not less than 95% (pass (passage of sieve) of 100M sieve, not less than 95%), more preferably not less than 97% (pass (passage of sieve) of 100M sieve, not less than 97%), for not more than 150 µm. The particle size is obtained, for example, by sieving using a standard sieve and measuring the weight of the granules remaining on the sieve.

The tablet of the present invention may further contain an additive conventionally used in the field of pharmaceutical preparation. Examples of the additive include excipients, disintegrant, lubricant, colorant, pH adjusting agent, surfactant, stabilizer, acidulant, flavor, coating base, coating additive and the like. Unless particularly indicated, these additives are used in an amount conventionally employed in the field of pharmaceutical preparation.

Preferable examples of the excipient include mannitol; starches such as corn starch, potato starch, wheat starch, rice starch, partly pregelatinized starch, pregelatinized starch, perforated starch and the like; anhydrous calcium phosphate; precipitated calcium carbonate; calcium silicate and the like.

Preferable examples of the disintegrant include corn starch, carboxymethylcellulose, carboxymethylcellulose calcium, carboxymethylstarch sodium, croscarmellose sodium (e.g., Ac-Di-Sol), crospovidone, low-substituted hydroxypropylcellulose (L-HPC), hydroxypropylstarch and the like.

As a disintegrant for the tablet of the present invention, croscarmellose sodium is preferable.

The amount of a disintegrant to be contained in one tablet of the present invention is preferably 1 - 10 weight%, more preferably 2 - 5 weight%.

Preferable examples of the lubricant include magnesium stearate, calcium stearate, talc, sucrose esters of fatty acids, sodium stearyl fumarate and the like.

As a lubricant for the tablet of the present invention, magnesium stearate is preferable.

The amount of a lubricant to be contained in one tablet of the present invention is preferably 0.3 - 2 weight%, more preferably 0.5 - 1.7 weight%.

Preferable examples of the colorant include food colors such as Food Color Yellow No. 5, Food Color Red No. 2, Food Color Blue No. 2 and the like, food lake colors, red ferric oxide (diiron trioxide), yellow ferric oxide and the like.

Preferable examples of the pH adjusting agent include citric acid or a salt thereof, phosphoric acid or a salt thereof, carbonic acid or a salt thereof, tartaric acid or a salt thereof, fumaric acid or a salt thereof, acetic acid or a salt thereof, amino acid or a salt thereof and the like.

Preferable examples of the surfactant include sodium lauryl sulfate, polysorbate 80, polyoxyethylene(160)polyoxypropylene(30)glycol and the like.

Preferable examples of the stabilizer include succinic acid, tartaric acid, citric acid, lactic acid, fumaric acid, malic acid, ascorbic acid, acetic acid, and acidic amino acid (e.g., glutamic acid, aspartic acid), inorganic salt (e.g., alkali metal salt, alkaline earth metal salt) of these acids, salts with inorganic base (e.g., ammonium) or organic base (e.g., meglumine) of these acids, salts with basic amino acid (e.g., arginine, lysin, ornithine)), water solvates thereof, solvent solvates thereof and the like.

Preferable examples of the acidulant include ascorbic acid, citric acid, tartaric acid, malic acid and the like.

Preferable examples of the flavor include menthol, peppermint oil, lemon oil, vanillin and the like.

Preferable examples of the coating base include sugar coating base, aqueous film coating base, enteric film coating base, sustained-release film coating base and the like.

Examples of the sugar coating base include sucrose. Furthermore, one or more kinds selected from talc, precipitated calcium carbonate, gelatin, gum arabic, pullulan, carnauba wax and the like may be used in combination.

Examples of the aqueous film coating base include cellulose polymers such as hydroxypropylcellulose [e.g., grades: L, SL, SL-T, SSL (trade name); Nippon Soda Co., Ltd.], hydroxypropylmethylcellulose [e.g., hypromellose2910, TC-5 (grades: MW, E, EW, R, RW) (trade name); Shin-Etsu Chemical Co., Ltd.]], hydroxyethylcellulose, methylhydroxyethylcellulose and the like; synthetic polymers such as polyvinyl acetal diethylaminoacetate, aminoalkylmethacrylate copolymer E [Eudragit E (trade name)], polyvinylpyrrolidone and the like; polysaccharides such as pullulan and the like, and the like.

Examples of the enteric film coating base include cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L [Eudragit L (trade name)], methacrylic acid copolymer LD [Eudragit L-30D55 (trade name)], methacrylic acid copolymer S [Eudragit S (trade name)] and the like; naturally occurring substances such as shellac and the like; and the like.

Examples of the sustained-release film coating base include cellulose polymers such as ethylcellulose and the like; acrylic acid polymers such as aminoalkyl methacrylate copolymer RS [Eudragit RS (trade name)], ethyl acrylate methacrylic acid methyl copolymer suspension [Eudragit NE (trade name)] and the like; and the like.

Preferable examples of the coating additive include light shielding agents such as titanium dioxide and the like, fluidizers such as talc and the like; colorants such as red ferric oxide (diiron trioxide), yellow ferric oxide and the like; plasticizers such as polyethylene glycol (e.g., macrogol 6000), triethyl citrate, castor oil, polysorbates and the like; organic acids such as citric acid, tartaric acid, malic acid, ascorbic acid and the like; and the like.

Two or more kinds of the above-mentioned additives may be mixed at an appropriate ratio and used.

The tablet of the present invention is preferably
(1) a tablet containing compound (A) or a salt thereof, a fluidizer, crystalline cellulose and a binder other than crystalline cellulose, wherein the content of compound (A) is 35 - 50 weight%;
(1A) a tablet containing compound (A) or a salt thereof, light anhydrous silicic acid, crystalline cellulose and hydroxypropylcellulose, wherein the content of compound (A) is more than 45 weight% to not more than 50 weight%;
(1B) a tablet containing compound (A) or a salt thereof, a fluidizer, crystalline cellulose and hydroxypropylcellulose, wherein the content of compound (A) is more than 45 weight% to not more than 50 weight%, and the content of the fluidizer is 0.1 - 1 weight% (preferably 0.1 - 0.6 weight%);
(1C) a tablet containing compound (A) or a salt thereof, light anhydrous silicic acid, crystalline cellulose and hydroxypropylcellulose, wherein the content of compound (A) is more than 45 weight% to not more than 50 weight% and the content of light anhydrous silicic acid is 0.1 - 1 weight% (preferably 0.1 - 0.6 weight%);
(2) a tablet containing compound (A) or a salt thereof, a fluidizer, crystalline cellulose and hydroxypropylcellulose, wherein the content of compound (A) is 35 - 50 weight%, the content of crystalline cellulose is 10 - 50 weight%, and the content of hydroxypropylcellulose is 3 - 10 weight%;
(2A) a tablet containing compound (A) or a salt thereof, light anhydrous silicic acid, crystalline cellulose and hydroxypropylcellulose, wherein the content of compound (A) is more than 45 weight% to not more than 50 weight%, the content of crystalline cellulose is 10 - 50 weight%, and the content of hydroxypropylcellulose is 3 - 10 weight%;
(2B) a tablet containing compound (A) or a salt thereof, a fluidizer, crystalline cellulose and hydroxypropylcellulose, wherein the content of compound (A) is more than 45 weight% to not more than 50 weight%, the content of the fluidizer is 0.1 - 1 weight% (preferably 0.1 - 0.6 weight%), and the content of crystalline cellulose is 10 - 50 weight%, and the content of hydroxypropylcellulose is 3 - 10 weight%;
(2C) a tablet containing compound (A) or a salt thereof, light anhydrous silicic acid, crystalline cellulose and hydroxypropylcellulose, wherein the content of compound (A) is more than 45 weight% to not more than 50 weight%, the content of light anhydrous silicic acid is 0.1 - 1 weight% (preferably 0.1 - 0.6 weight%), the content of crystalline cellulose is 10 - 50 weight%, and the content of hydroxypropylcellulose is 3 - 10 weight%;
(3) a tablet containing compound (A) or a salt thereof, a fluidizer, crystalline cellulose, a binder other than crystalline cellulose, a disintegrant and a lubricant, wherein the content of compound (A) is 35 - 50 weight%;
(3A) a tablet containing compound (A) or a salt thereof, light anhydrous silicic acid, crystalline cellulose, hydroxypropylcellulose, croscarmellose sodium and magnesium stearate, wherein the content of compound (A) is more than 45 weight% to not more than 50 weight%;
(3B) a tablet containing compound (A) or a salt thereof, a fluidizer, crystalline cellulose, hydroxypropylcellulose, a disintegrant and a lubricant, wherein the content of compound (A) is more than 45 weight% to not more than 50 weight%, the content of a fluidizer is 0.1 - 1 weight% (preferably 0.1 - 0.6 wight%), the content of crystalline cellulose is 10 - 50 weight%, and the content of hydroxypropylcellulose is 3 - 10 weight%;
(3C) a tablet containing compound (A) or a salt thereof, light anhydrous silicic acid, crystalline cellulose, hydroxypropylcellulose, a disintegrant and a lubricant, wherein the content of compound (A) is more than 45 weight% to not more than 50 weight%, the content of light anhydrous silicic acid is 0.1 - 1 weight% (preferably 0.1 - 0.6 weight%), the content of crystalline cellulose is 10 - 50 weight%, and the content of hydroxypropylcellulose is 3 - 10 weight%;
(3D) a tablet containing compound (A) or a salt thereof, light anhydrous silicic acid, crystalline cellulose, hydroxypropylcellulose, a disintegrant and a lubricant, wherein the content of compound (A) is more than 45 weight% to not more than 50 weight%, the content of light anhydrous silicic acid is 0.1 - 1 weight% (preferably 0.1 - 0.6 weight%), the content of crystalline cellulose is 10 - 50 weight%, the content of hydroxypropylcellulose is 3 - 10 weight%, and the content of the disintegrant is 1 - 10 weight%;
(3E) a tablet containing compound (A) or a salt thereof, light anhydrous silicic acid, crystalline cellulose, hydroxypropylcellulose, a disintegrant and a lubricant, wherein the content of compound (A) is more than 45 weight% to not more than 50 weight%, the content of light anhydrous silicic acid is 0.1 - 1 weight% (preferably 0.1 - 0.6 weight%), the content of crystalline cellulose is 10 - 50 weight%, the content of hydroxypropylcellulose is 3 - 10 weight%, the content of disintegrant is 1 - 10 weight%), and the content of the lubricant is 0.3 - 2 weight%;
   and the like.

The tablet of the present invention includes sublingual tablet, orally disintegrating tablet and the like, and the shape of the tablet may be any of round, caplet, oblong and the like.

The tablet of the present invention can be produced by formulating with further additives as necessary and according to a method conventionally used in the technical field of pharmaceutical preparation.

Here, the further additive includes those similar to the aforementioned additives.

Two or more kinds of the above-mentioned further additives may be mixed at an appropriate ratio and used.

The tablet of the present invention may be film-coated to improve dosability, strength of preparation and the like.

The hardness of the tablet before film coating is preferably 2.1 - 5 N/mm², more preferably 2.3 - 4 N/mm², to prevent breakage of tablet surface and crack of tablet that occur during the production step. N/mm² shows hardness per unit fracture area of the tablet. For example, when the tablet hardness is 150N and the fracture area is 60 mm², the tablet hardness is 2.5 N/mm².

Preferable examples of the coating base and coating additive used for film coating include those similar to the coating base and coating additive used for the aforementioned additives.

When the tablet of the present invention is film-coated, a film coating layer is generally formed in the proportion of 1 - 10 parts by weight, preferably 2 - 6 parts by weight, per 100 parts by weight of the tablet.

The tablet of the present invention is produced by appropriately combining operations such as granulation, mixing, tableting (compression molding), coating and the like.

The granulation step is performed by using a granulator such as mixer granulator, fluid granulator, dry granulator and the like.

The mixing step is performed by, for example, using a mixer such as V-type mixer, tumbler mixer and the like.

The tableting (compression molding) step is performed by, for example, using a single punch tableting machine, rotary tableting machine etc. and tableting generally under a pressure of 0.3 - 35 kN/cm².

In addition, the coating is performed by, for example, using a film coating apparatus. As the coating base, for example, sugar coating base, aqueous film coating base, enteric film coating base, sustained-release film coating base and the like are used.

Examples of the sugar coating base, aqueous film coating base, enteric film coating base, sustained-release film coating base, and coating additive include those similar to the aforementioned additives.

Two or more kinds of the above-mentioned coating bases may be mixed at an appropriate ratio and used. Moreover, coating additive may be used for coating.

In a preferable embodiment, the tablet of the present invention can be produced according to the following production step. Each starting material for the following production step is used in such an amount that achieves the above-mentioned content in the finally-obtained tablet.
1) Compound (A) or a salt thereof, a fluidizer (e.g., light anhydrous silicic acid) and, where necessary, other additives (e.g., binder, disintegrant, lubricant) are mixed by an appropriate blending machine, the mixture is granulated by a roller compacter and the obtained granulation product is cracked to give a sieved powder.
2) To the sieved powder were added a binder (e.g., crystalline cellulose, hydroxypropylcellulose) and, where necessary, other additives (e.g., lubricant), and they are mixed to give granules for tableting.
3) The granules are tableted by a tableting machine to give an uncoated tablet.
4) When desired, a film coating solution is sprayed on the obtained uncoated tablet in, for example, a film coating machine to give a film-coated tablet.

In another preferable embodiment, the tablet of the present invention is
(1) a tablet containing the following (a1), wherein the content of compound (A) is 35 - 50 weight% (preferably, more than 45 weight% to not more than 50 weight%):
   (a1) a granule containing compound (A) or a salt thereof, and a fluidizer (preferably, light anhydrous silicic acid),
(2) a tablet containing the following (a1) and (b1), wherein the content of compound (A) is 35 - 50 weight% (preferably, more than 45 weight% to not more than 50 wight%) :
   (a1) a granule containing compound (A) or a salt thereof, and a fluidizer (preferably, light anhydrous silicic acid);
   (b1) a tableting aid containing a binder (preferably, crystalline cellulose and hydroxypropylcellulose),
(3) a tablet containing the following (a1) and (b2), wherein the content of compound (A) is 35 - 50 weight% (preferably, more than 45 weight% to not more than 50 weight%):
   (a1) a granule containing compound (A) or a salt thereof, and a fluidizer (preferably, light anhydrous silicic acid);
   (b2) a tableting aid containing crystalline cellulose and a binder other than crystalline cellulose (preferably, hydroxypropylcellulose),
(4) a tablet containing the following (a2) and (b3), wherein the content of compound (A) is 35 - 50 weight% (preferably, more than 45 weight% to not more than 50 weight%):
   (a2) a granule containing compound (A) or a salt thereof, a fluidizer (preferably, light anhydrous silicic acid), and a binder (preferably, crystalline cellulose);
   (b3) a tableting aid containing crystalline cellulose, a binder other than crystalline cellulose (preferably, hydroxypropylcellulose), and a lubricant (preferably, magnesium stearate),
(5) a tablet containing the following (a3) and (b3), wherein the content of compound (A) is 35 - 50 weight% (preferably, more than 45 weight% to not more than 50 weight%):
   (a3) a granule containing compound (A) or a salt thereof, a fluidizer (preferably, light anhydrous silicic acid), a binder (preferably, crystalline cellulose), a disintegrant (preferably, croscarmellose sodium) and a lubricant (preferably, magnesium stearate);
   (b3) a tableting aid containing crystalline cellulose, a binder other than crystalline cellulose (preferably, hydroxypropylcellulose), and a lubricant (preferably, magnesium stearate),
      and the like.

In this embodiment, the tablet of the present invention contains preferably 75 - 100 weight%, more preferably 75 - 95 weight%, further more preferably 80 - 90 weight%, of granules.

Here, the "granule" means a granule having a nearly uniform shape and size, which is obtained by granulating a starting material of powder, bulk, solution, molten liquid and the like by a wet granulation method, dry granulation method, heating granulation method (preferably, dry granulation method) and the like.

The granule in this embodiment generally has a particle size of not more than 20% for not less than 1000 µm and not more than 65% for not more than 150 µm (on (remaining on the sieve) of 16M sieve: not more than 20%; pass (passage of sieve) of 100M sieve, not more than 65%), preferably not more than 5% for not less than 1000 µm, not more than 40% for not more than 150 µm (on (remaining on the sieve) of 16M sieve: not more than 5%; pass (passage of sieve) of 100M sieve, not more than 40%) The particle size here is obtained, for example, by sieving using a standard sieve and measuring the weight of the granules remaining on the sieve.

The granule may have shape and size that have changed during the formulation process (e.g., tableting step) to obtain the tablet of the present invention.

The granule contains an additive conventionally used in the field of pharmaceutical preparation. Examples of the additive include those mentioned above and the like. Unless particularly indicated, these additives are used in an amount conventionally employed in the field of pharmaceutical preparation. The granule may contain two or more kinds of these additives at an appropriate ratio.

The granule is preferably
(a1) a granule containing compound (A) or a salt thereof, and a fluidizer (preferably, light anhydrous silicic acid),
(a2) a granule containing compound (A) or a salt thereof, a fluidizer (preferably, light anhydrous silicic acid), and a binder (preferably, crystalline cellulose),
(a3) a granule containing compound (A) or a salt thereof, a fluidizer (preferably, light anhydrous silicic acid), a binder (preferably, crystalline cellulose), a disintegrant (preferably, croscarmellose sodium) and a lubricant (preferably, magnesium stearate),
   and the like.

In this embodiment, the amount of compound (A) contained in the granules in the tablet of the present invention is 40 - 60 weight%, preferably 45 - 60 weight%, more preferably 50 - 55 weight%.

The amount of the fluidizer contained in the granule is preferably 0.1 - 1.2 weight%, more preferably 0.1 - 0.8 weight%.

The amount of the binder contained in the granule is preferably 20 - 50 weight%, more preferably 20 - 30 weight%.

The amount of the disintegrant contained in the granule is preferably 1 - 10 weight%, more preferably 2 - 5 weight%.

The amount of the lubricant contained in the granule is preferably 0.1 - 2 weight%, more preferably 0.1 - 1 weight%.

In addition, the "tableting aid" means a component to be mixed with a granule containing compound (A) or a salt thereof, before the tableting step in the production of a tablet. The component may contain one or more kinds of the additives to be mentioned later.

The tableting aid contains an additive conventionally used in the pharmaceutical field. Examples of the additive include the above-mentioned additives and the like. Unless particularly specified, these additives are used in an amount conventionally used in the pharmaceutical field. The tableting aid may contain two or more kinds of these additives at an appropriate ratio.

The tableting aid in this embodiment is preferably
(b1) a tableting aid containing a binder (preferably, crystalline cellulose, hydroxypropylcellulose),
(b2) a tableting aid containing crystalline cellulose and a binder other than crystalline cellulose (preferably, hydroxypropylcellulose),
(b3) a tableting aid containing crystalline cellulose, a binder other than crystalline cellulose (preferably, hydroxypropylcellulose), and a lubricant(preferably, magnesium stearate),
   and the like.

In this embodiment, the amount of the binder contained in the tableting aid in the tablet of the present invention is preferably 85 - 95 weight%, more preferably 90 - 95 weight%.

When the tableting aid contains two or more kinds of binders, the combination of the binders is preferably crystalline cellulose and a binder other than crystalline cellulose (hydroxypropylcellulose, hydroxypropylmethylcellulose, povidone), and more preferably, crystalline cellulose and hydroxypropylcellulose. The amount of the binder to be contained in the tableting aid is preferably 50 - 70 weight% for crystalline cellulose, and 20 - 40 weight% for a binder other than crystalline cellulose (preferably, hydroxypropylcellulose), more preferably, 55 - 65 weight% for crystalline cellulose, and 25 - 35 weight% for a binder other than crystalline cellulose (preferably, hydroxypropylcellulose).

The amount of the lubricant to be contained in the tableting aid is preferably 5 - 15 weight%, more preferably 5 - 10 weight%.

In this embodiment, the tablet of the present invention generally contains preferably 75 - 95 weight%, more preferably 80 - 90 weight%, of the granule and preferably 5 - 25 weight%, more preferably 10 - 20 weight%, of the tableting aid.

In this embodiment, the tablet of the present invention can be produced by mixing the granule and a tableting aid, and tableting the mixture.

In a preferable embodiment, a tablet can be produced according to the following production step. Each starting material for the following production step is used in such an amount that achieves the above-mentioned content in the finally-obtained tablet.
1) As for granule, for example, compound (A) or a salt thereof, a fluidizer and, where necessary, other additives (e.g., binder, disintegrant, lubricant) are mixed in an appropriate blending machine, the mixture is granulated by a roller compacter and the obtained granulation product is cracked to give a sieved powder.
2) To the sieved powder was added a tableting aid (e.g., binder, lubricant), and they are mixed to give granules for tableting.
3) The granules are tableted by a tableting machine to give an uncoated tablet.
4) When desired, a film coating solution is sprayed on the obtained uncoated tablet in, for example, a film coating machine to give a film-coated tablet.

The tablet of the present invention may be film-coated to improve dosability, strength of preparation and the like. In addition, the tablet may be filled in a capsule (e.g., gelatin capsule) to give a capsule.

The tablet of the present invention may be stamped or printed with marks or letters for discrimination, or have a separating line for dividing the tablet.

In the above-mentioned production steps, the operations such as mixing, tableting, coating and the like in the aforementioned production step are performed according to a method conventionally used in the technical field of pharmaceutical preparations.

The tablet of the present invention is low toxic and can be safely administered orally or parenterally to a mammal (e.g., mouse, rat, rabbit, cat, dog, bovine, horse, monkey, human).

The tablet of the present invention is useful for the prophylaxis or treatment of, for example, diabetes [e.g., type 1 diabetes, type 2 diabetes, type 1.5 diabetes (LADA (Latent Autoimmune Diabetes in Adults)), gestational diabetes, diabetes with impaired insulin secretion, obese diabetes, impaired glucose tolerance (IGT (Impaired Glucose Tolerance)), IFG (Impaired Fasting Glucose), IFG (Impaired Fasting Glycaemia)], diabetic complications [e.g., neuropathy, nephropathy, retinopathy, cataract, macroangiopathy, arteriosclerosis, osteopenia, hyperosmolar diabetic coma, infections (e.g., respiratory infection, urinary tract infection, gastrointestinal infection, dermal soft tissue infections, inferior limb infection), diabetic gangrene, xerostomia, hypacusis, cerebrovascular disorder, peripheral blood circulation disorder], obesity, hyperlipidemia (e.g., hypertriglyceridemia, hypercholesterolemia, hypoHDL-emia, postprandial hyperlipemia), arteriosclerosis (e.g., atherosclerosis), hypertension, myocardial infarction, angina pectoris, cerebrovascular disorder (e.g., cerebral infarction, cerebral apoplexy), insulin resistance syndrome, syndrome X, dysmetabolic syndrome) and the like.

In addition, the tablet of the present invention is also useful for secondary prevention of the above-mentioned various diseases (e.g., secondary prevention of cardiovascular event such as myocardial infarction and the like) or suppression of progression [e.g., suppression of progression from impaired glucose tolerance to diabetes; suppression of progression from diabetes to diabetic complications (preferably diabetic neuropathy, diabetic nephropathy, diabetic retinopathy, arteriosclerosis)].

The dose of the tablet of the present invention may be an effective amount of compound (A) or a salt thereof to be contained as a pharmaceutically active ingredient. For example, for administration to an adult (body weight 60 kg) at intervals beyond one day (e.g., once per 3 days - one week), the dose is generally 1 mg - 500 mg, preferably 1 mg - 400 mg, more preferably 25 - 250 mg, still more preferably 50 mg - 200 mg, per one administration based on compound (A) free form. For example, when a single dose is 50 mg based on compound (A) free form, a tablet having a total weight per tablet of 150 mg or less can be provided.

Since the tablet of the present invention contains a high dose of compound (A) or a salt thereof and of a size permitting easy administration, it is preferable for administration of a high dose of compound (A) or a salt thereof. For example, it is preferable for administration at intervals beyond one day (preferably, once per week), and can be provided as a dosage form of once per 3 days or once per 2 weeks (preferably, dosage form of once per week).

While the size of the tablet of the present invention varies depending on the shape of the tablet (round, caplet, oblong etc.), it only needs to be a size easily taken by patients. For example, when the shape of the tablet is oblong, the size of the tablet is preferably longest diameter: not less than 4 mm and not more than 9 mm, more preferably not less than 5 mm and not more than 8 mm. When the shape of the tablet is round, the size of the tablet is preferably diameter: not less than 4 mm and not more than 9 mm, more preferably not less than 5 mm and not more than 8 mm.

Particularly preferable embodiment of the tablet of the present invention includes
"a tablet containing 50 mg of compound (A) (free form) per tablet";
"a tablet containing 100 mg of compound (A) (free form) per tablet"; and
"a tablet containing 200 mg of compound (A) (free form) per tablet".

Compound (A) or a salt thereof can be used in combination with one or more kinds of other drugs (hereinafter sometimes to be referred to as "concomitant drug".

AS a specific example, compound (A) or a salt thereof can be used in combination with one or more pharmaceutical agents selected from a therapeutic agent for diabetes, a therapeutic agent for diabetic complications, a therapeutic agent for hyperlipidemia, an antihypertensive agent, an antiobesitic agent, a diuretic, an antithrombotic agent and the like (concomitant drug).

Examples of the therapeutic agent for diabetes include insulin preparations (e.g., animal insulin preparation extracted from the pancreas of bovine or swine; human insulin preparation synthesized by genetic engineering using Escherichia coli or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), metaglidasen, AMG-131, balaglitazone, MBX-2044, rivoglitazone, aleglitazar, chiglitazar, lobeglitazon, PLX-204, PN-2034, GFT-505, THR-0921, compound described in WO2007/013694 WO2007/018314, WO2008/093639 or WO2008/099794), α-glucosidase inhibitors (e.g., voglibose, acarbose, miglitol, emiglitate), biguanides (e.g., metformin, buformin or salts thereof (e.g., hydrochloride, fumarate, succinate)), insulin secretagogue (sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof), dipeptidyl peptidase IV inhibitors (e.g., alogliptin or a salt thereof (preferably, benzoate), vildagliptin, sitagliptin, saxagliptin, BI1356, GRC8200, MP-513, PF-00734200, PHX1149, SK-0403, ALS2-0426, TA-6666, TS-021, KRP-104), β3 agonists (e.g., N-5984), GPR40 agonists (e.g., compound described in WO2004/041266 WO2004/106276, WO2005/063729, WO2005/063725, WO2005/087710, WO2005/095338, WO2007/013689 or WO2008/001931), GLP-1 receptor agonists (e.g., GLP-1, GLP-1MR agent, liraglutide, exenatide, AVE-0010, BIM-51077, Aib(8,35)hGLP-1(7,37)NH2, CJC-1131, Albiglutide), amylin agonists (e.g., pramlintide), phosphotyrosine phosphatase inhibitors (e.g., sodium vanadate), gluconeogenesis inhibitors (e.g., glycogen phosphorylase inhibitor, glucose-6-phosphatase inhibitor, glucagon antagonist, FBPase inhibitor), SGLT2 (sodium-glucose cotransporter 2) inhibitors (e.g., depagliflozin, AVE2268, TS-033, YM543, TA-7284, remogliflozin, ASP1941), SGLT1 inhibitors, 11β-hydroxysteroid dehydrogenase inhibitors (e.g., BVT-3498, INCB-13739), adiponectin or agonist thereof, IKK inhibitors (e.g., AS-2868), leptin resistance improving drugs, somatostatin receptor agonists, glucokinase activators (e.g., piragliatin, AZD1656, AZD6370, TTP-355, compound described in WO2006/112549, WO2007/028135, WO2008/047821, WO2008/050821, WO2008/136428 or WO2008/156757), GIP (Glucose-dependent insulinotropic peptide), GPR119 agonists (e.g., PSN821), FGF21, FGF analogue and the like.

Examples of the therapeutic agents for diabetic complications include aldose reductase inhibitors (e.g., tolrestat, epalrestat, zopolrestat, fidarestat, CT-112, ranirestat (AS-3201), Lidorestat), neurotrophic factors and increasing drugs thereof (e.g., NGF, NT-3, BDNF, neurotrophin production/secretion promoting agent described in WO01/14372 (e.g., 4-(4-chlorophenyl)-2-(2-methyl-1-imidazolyl)-5-[3-(2-methylphenoxy)propyl]oxazole), compound described in WO2004/039365), PKC inhibitors (e.g., ruboxistaurin mesylate), AGE inhibitors (e.g., ALT946, N-phenacylthiazolium bromide (ALT766), EXO-226, pyridorin, pyridoxamine), GABA receptor agonists (e.g., gabapentin, Pregabalin), serotonin noradrenaline reuptake inhibitors (e.g., duloxetine), sodium channel inhibitors (e.g., Lacosamide), active oxygen scavengers (e.g., thioctic acid), cerebral vasodilators (e.g., tiapuride, mexiletine), somatostatin receptor agonists (e.g., BIM23190), apoptosis signal regulating kinase-1 (ASK-1) inhibitor and the like.

Examples of the therapeutic agent for hyperlipidemia include HMG-CoA reductase inhibitors (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compound described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), anion-exchange resins (e.g., colestyramine), probucol, nicotinic acid drugs (e.g., nicomol, niceritrol, niaspan), ethyl icosapentate, phytosterol (e.g., soysterol, gamma oryzanol), cholesterol absorption inhibitors (e.g., Zetia), CETP inhibitors (e.g., dalcetrapib, anacetrapib), ω-3 fatty acid preparation (e.g., ω-3-acid ethyl esters 90) and the like.

Examples of the antihypertensive agent include angiotensin converting enzyme inhibitors (e.g., captopril, enalapril, delapril etc.), angiotensin II antagonists (e.g., candesartan cilexetil, candesartan, losartan, potassium losartan, eprosartan, valsartan, telmisartan, irbesartan, tasosartan, olmesartan, olmesartan medoxomil, azilsartan, azilsartan medoxomil etc.), calcium antagonists (e.g., manidipine, nifedipine, amlodipine, efonidipine, nicardipine, amlodipine, cilnidipine etc.), β-blockers (e.g., metoprolol, atenolol, propranolol, carvedilol, pindolol etc.), clonidine and the like.

Examples of the antiobesitic agent include monoamine uptake inhibitors (e.g., phentermine, sibutramine, mazindol, fluoxetine, tesofensine), serotonin 2C receptor agonists (e.g., lorcaserin), serotonin 6 receptor antagonists, histamine H3 receptor, GABA modulating agents (e.g., topiramate), neuropeptide Y antagonists (e.g., velneperit), cannabinoid receptor antagonists (e.g., rimonabant, Taranabant), ghrelin antagonists, ghrelin receptor antagonists, ghrelin acylated enzyme inhibitor, opioid receptor antagonists (e.g., GSK-1521498), orexin receptor antagonist, melanocortin 4 receptor agonist, 11β-hydroxysteroid dehydrogenase inhibitors (e.g., AZD-4017), pancreatic lipase inhibitors (e.g., orlistat, cetilistat), β3 agonists (e.g., N-5984), diacylglycerol acyltransferase 1 (DGAT1) inhibitor, acetyl-CoA carboxylase (ACC) inhibitor, stearoyl CoA desaturase inhibitor, microsomal triglyceride transfer protein inhibitors (e.g., R-256918), Naglucose cotransport carrier inhibitors (e.g., JNJ-28431754, remogliflozine), NFκB inhibitors (e.g., HE-3286), PPAR agonists (e.g., GFT-505, DRF-11605), phosphotyrosine phosphatase inhibitors (e.g., sodium vanadate, Trodusquemin), GPR119 agonists (e.g., PSN-821), glucokinase activators (e.g., AZD-1656), leptin, leptin derivatives (e.g., metreleptin), CNTF (ciliary neurotrophic factor), BDNF (brain-derived neurotrophic factor), cholecystokinin agonist, glucagon-like peptide-1 (GLP-1) preparations (e.g., animal GLP-1 preparation extracted from the pancreas of bovine or swine; human GLP-1 preparation synthesized by genetic engineering using Escherichia coli or yeast; GLP-1 fragment or derivative (e.g., exenatide, liraglutide)), amylin preparations (e.g., pramlintide, AC-2307), neuropeptide Y agonists (e.g., PYY3-36, derivative of PYY3-36, obineptide TM-30339, TM-30335), oxyntomodulin preparation: FGF21 preparations (e.g., animal FGF21 preparation extracted from the pancreas of bovine or swine; human FGF21 preparation synthesized by genetic engineering using Escherichia coli or yeast; FGF21 fragment or derivative), anorexigenic agents (e.g., P-57) and the like.

Examples of the diuretic include xanthine derivatives (e.g., theobromine sodium salicylate, theobromine calcium salicylate etc.), thiazide preparations (e.g., ethiazide, cyclopenthiazide, trichloromethyazide, hydrochlorothiazide, hydroflumethiazide, benzylhydrochlorothiazide, penfluthiazide, poly5thiazide, methyclothiazide etc.), antialdosterone preparations (e.g., spironolactone, triamterene etc.), carbonic anhydrase inhibitors (e.g., acetazolamide etc.), chlorobenzenesulfonamide agents (e.g., chlortalidone, mefruside, indapamide etc.), azosemide, isosorbide, ethacrynic acid, piretanide, bumetanide, furosemide and the like, and the like.

Examples of the antithrombotic agent include heparin (e.g., heparin sodium, heparin calcium, enoxaparin sodium, dalteparin sodium), warfarin (e.g., warfarin potassium), anti-thrombin drugs (e.g., aragatroban, dabigatran), FXa inhibitors (e.g., rivaroxaban, apixaban, edoxaban, YM150, compound described in WO02/06234, WO2004/048363, WO2005/030740, WO2005/058823 or WO2005/113504), thrombolytic agents (e.g., urokinase, tisokinase, alteplase, nateplase, monteplase, pamiteplase), platelet aggregation inhibitors (e.g., ticlopidine hydrochloride, clopidogrel, prasugrel, E5555, SHC530348, cilostazol, ethyl icosapentate, beraprost sodium, sarpogrelate hydrochloride), and the like.

Among the above-mentioned concomitant drugs, insulin sensitizers (preferably, pioglitazone hydrochloride), insulin preparation, α-glucosidase inhibitors (preferably, voglibose, acarbose), biguanides (preferably, metformin hydrochloride), sulfonylurea (preferably, glimepiride) and the like are preferable.

When the tablet of the present invention and a concomitant drug are used in combination, the administration time of these is not limited, and they can be administered simultaneously to an administration subject, or may be administered in a staggered manner.

In addition, the tablet of the present invention and the concomitant drug may be administered as separate preparations to an administration subject, or may be administered as a single preparation containing the tablet of the present invention and the concomitant drug.

The dose of the concomitant drug can be appropriately determined based on the clinically employed dose of each drug. In addition, the mixing ratio of the tablet of the present invention and the concomitant drug can be appropriately determined according to the administration subject, administration route, target disease, condition, combination and the like. For example, when the administration subject is a human, the concomitant drug may be used in an amount of 0.01 to 100 parts by weight per 1 part by weight of the tablet of the present invention.

Use of the concomitant drug in this way provides superior effects such as 1) enhanced action of compound (A) (or a salt thereof) or the concomitant drug (synergistic effect of the actions of the pharmaceutical agents), 2) reduced dose of compound (A) (or a salt thereof) or the combination drug (effect of reduction of dose of pharmaceutical agents as compared to single drug administration), 3) reduced secondary action of compound (A) (or a salt thereof) or the concomitant drug, and the like.

The present invention is explained in more detail in the following by referring to Examples, Reference Examples and Experimental Examples, which are not to be construed as limitative.

As additives for pharmaceutical preparations in the following Examples and Experimental Examples, the Japanese Pharmacopoeia 15th edition, the Japanese Pharmacopoeia Japanese Pharmaceutical Codex or Japanese Pharmaceutical Excipients 2003 compatible products were used.

### Example 1

According to the formulation of Table 1, succinate (monosuccinate) of compound (A), crystalline cellulose (PH-302), croscarmellose sodium, magnesium stearate and light anhydrous silicic acid were uniformly mixed in a mixer (vertical granulator 50 L, Powrex Corporation), and granulated by a roller compacter (Alexander). The obtained granulation product was sieved by a granulator (power mill P-3, Showa Kako Corporation) to give a sieved powder. To the sieved powder were added crystalline cellulose (KG-802), hydroxypropylcellulose (HPC-L100M; Nippon Soda Co., Ltd.) and magnesium stearate, and they were mixed in a blending machine (tumbler 60 L, Showa Kako Corporation) to give granules for tableting. The granules were tableted in a rotary tableting machine (AQUARIOUS3-A, Kikusui Seisakusho Ltd.) with a 8×4.5 mm punch to a weight of 110 mg to give an uncoated tablet. Separately, titanium oxide, diiron trioxide, yellow ferric oxide and talc were dispersed in an aqueous hydroxypropylmethylcellulose (TC-5RW; Shin-Etsu Chemical Co., Ltd.) solution to prepare a film coating solution. Onto the above-mentioned uncoated tablet was sprayed the aforementioned coating solution in a film coating machine (DRIACOATER DRC500, Powrex Corporation) to give 30,000 film-coated tablets containing 50 mg of compound (A) (free form) per tablet.

**Table 1**

| ingredients | compound (A) free form 50 mg tablet |
|---|---|
| | mg/tablet |
| (granule) | |
| | |
| succinate of compound (A) | 66.5 |
| crystalline cellulose (PH-302) | 23.35 |
| croscarmellose sodium | 3 |
| magnesium stearate | 0.33 |
| light anhydrous silicic acid | 0.5 |
| (tableting aid) | |
| | |
| crystalline cellulose (KG-802) | 10 |
| magnesium stearate | 1.32 |
| hydroxypropylcellulose | 5 |
| (film coating) | |
| | |
| hypromellose2910 | 3.12 |
| titanium oxide | 0.4 |
| diiron trioxide | 0.004 |
| yellow ferric oxide | 0.016 |
| talc | 0.48 |
| total weight | 114.02 |

### Example 2

According to the formulation of Table 2, succinate (monosuccinate) of compound (A), crystalline cellulose (PH-302), croscarmellose sodium, magnesium stearate and light anhydrous silicic acid were uniformly mixed in a mixer (vertical granulator 50 L, Powrex Corporation), and granulated by a roller compacter (Alexander). The obtained granulation product was sieved by a granulator (power mill P-3, Showa Kako Corporation) to give a sieved powder. To the sieved powder were added crystalline cellulose (KG-802), hydroxypropylcellulose (HPC-L100M; Nippon Soda Co., Ltd.) and magnesium stearate, and they were mixed in a blending machine (tumbler 60 L, Showa Kako Corporation) to give granules for tableting. The granules were tableted in a rotary tableting machine (AQUARIOUS3-A, Kikusui Seisakusho Ltd.) with a 11×6 mm punch to a weight of 220 mg to give an uncoated tablet. Separately, titanium oxide, diiron trioxide, yellow ferric oxide and talc were dispersed in an aqueous hydroxypropylmethylcellulose (TC-5RW; Shin-Etsu Chemical Co., Ltd.) solution to prepare a film coating solution. Onto the above-mentioned uncoated tablet was sprayed the aforementioned coating solution in a film coating machine (DRIACOATER DRC500, Powrex Corporation) to give 15,000 film-coated tablets containing 100 mg of compound (A) (free form) per tablet.

**Table 2**

| ingredient | compound (A) free form 100 mg tablet |
|---|---|
| | mg/tablet |
| (granule) | |
| | |
| succinate of compound (A) | 133 |
| crystalline cellulose (PH-302) | 46.7 |
| croscarmellose sodium | 6 |
| magnesium stearate | 0.66 |
| Light anhydrous silicic acid | 1 |
| (tableting aid) | |
| | |
| crystalline cellulose (KG-802) | 20 |
| magnesium stearate | 2.64 |
| hydroxypropylcellulose | 10 |
| (film coating) | |
| | |
| hypromellose2910 | 6.24 |
| titanium oxide | 0.8 |
| diiron trioxide | 0.008 |
| yellow ferric oxide | 0.032 |
| talc | 0.96 |
| total weight | 228.04 |

### Example 3

According to the formulation of Table 3, succinate (monosuccinate) of compound (A), crystalline cellulose (PH-302), croscarmellose sodium, magnesium stearate and light anhydrous silicic acid were uniformly mixed in a plastic bag, and granulated by a roller compacter (Alexander). The obtained granulation product was sieved by a granulator (power mill P-3, Showa Kako Corporation) to give a sieved powder. To the sieved powder were added crystalline cellulose (KG-802), hydroxypropylcellulose (HPC-L100M; Nippon Soda Co., Ltd.) and magnesium stearate, and they were mixed in a plastic bag to give granules for tableting. The granules were tableted in a rotary tableting machine (AQUARIOUS19K, Kikusui Seisakusho Ltd.) with a 13×8 mm punch to a weight of 440 mg to give an uncoated tablet. Separately, titanium oxide, diiron trioxide, yellow ferric oxide and talc were dispersed in an aqueous hydroxypropylmethylcellulose (TC-5RW; Shin-Etsu Chemical Co., Ltd.) solution to prepare a film coating solution. Onto the above-mentioned uncoated tablet was sprayed the aforementioned coating solution in a film coating machine (high coater mini, Freund Corporation) to give 700 film-coated tablets containing 200 mg of compound (A) (free form) per tablet.

**Table 3**

| ingredient | compound (A) free form 200 mg tablet |
|---|---|
| | mg/tablet |
| (granule) | |
| | |
| succinate of compound (A) | 266 |
| crystalline cellulose (PH-302) | 93.4 |
| croscarmellose sodium | 12 |
| magnesium stearate | 1.32 |
| light anhydrous silicic acid | 2 |
| (tableting aid) | |
| | |
| crystalline cellulose (KG-802) | 40 |
| magnesium stearate | 5.28 |
| hydroxypropylcellulose | 20 |
| (film coating) | |
| | |
| hypromellose2910 | 12.48 |
| titanium oxide | 1.6 |
| diiron trioxide | 0.016 |
| yellow ferric oxide | 0.064 |
| talc | 1.92 |
| total weight | 456.08 |

### Reference Example 1

In a fluidized-bed granulation dryer (FD-5S, Powrex Corporation) and according to the formulation of Table 4, succinate (monosuccinate) of compound (A), mannitol and corn starch were uniformly mixed, and the mixture was granulated while spraying an aqueous solution of hydroxypropylcellulose (HPC-L; Japan Nippon Soda Co., Ltd.) thereon, and dried in the same machine. The obtained granulation product was sieved by a granulator (power mill P-3, Showa Kako Corporation) to give a sieved powder. To the sieved powder were added croscarmellose sodium, crystalline cellulose (KG-802) and magnesium stearate, and they were mixed in a blending machine (tumbler 15 L, Showa Kako Corporation) to give granules for tableting. The granules were tableted in a rotary tableting machine (CORRECT 12HUK, Kikusui Seisakusho Ltd.) with a 6.5 mmϕ punch to a weight of 121 mg to give an uncoated tablet. Separately, titanium oxide, yellow ferric oxide and talc were dispersed in an aqueous hypromellose (TC-5R; Shin-Etsu Chemical Co., Ltd.) solution to prepare a film coating solution. Onto the above-mentioned uncoated tablet was sprayed the aforementioned coating solution in a film coating machine (DRIACOATER DRC500, Powrex Corporation) to give 26,000 film-coated tablets containing 25 mg of compound (A) (free form) per tablet.

**[Table 4]**

| ingredient | compound (A) free form 25 mg tablet |
|---|---|
| | mg/tablet |
| (granule) | |
| | |
| succinate of compound (A) | 33.25 |
| mannitol | 37.5 |
| corn starch | 29.35 |
| hydroxypropylcellulose | 3.3 |
| (tableting aid) | |
| | |
| croscarmellose sodium | 5.5 |
| crystalline cellulose (KG-802) | 11 |
| magnesium stearate | 1.1 |
| (film coating) | |
| hypromellose | 3.9 |
| titanium oxide | 0.5 |
| diiron trioxide | 0.005 |
| yellow ferric oxide | 0.02 |
| talc | 0.6 |
| total weight | 126.025 |

### Experimental Example 1

### (1) Production of comparison formulation 1A

Succinate (monosuccinate) of compound (A) (333 g), crystalline cellulose (PH-302, 119 g), croscarmellose sodium (15 g) and magnesium stearate (1.65 g) were uniformly mixed in a vertical granulator (Powrex Corporation) and granulated by a roller compacter (Alexander Verlag). As a result, blocking of powder caused fluidity failure, and the object granulation product could not be obtained.

### (2) Production of comparison formulation 1B

Succinate (monosuccinate) of compound (A) (200 g), crystalline cellulose (PH-302, 252 g), croscarmellose sodium (15 g) and magnesium stearate (1.65 g) were uniformly mixed in a vertical granulator (Powrex Corporation), and granulated by a roller compacter (Alexander Verlag) to give a granulation product (462 g). During the production of comparison formulation 1B, blocking and fluidity failure were not observed.

### (3) Production of formulation 1A

In formulation 1A, succinate (monosuccinate) of compound (A) (333 g), crystalline cellulose (PH-302, 117 g), croscarmellose sodium (15 g), magnesium stearate (1.65 g) and light anhydrous silicic acid (2.5 g) were uniformly mixed in a vertical granulator (Powrex Corporation). The obtained mixture (450 g) was processed by a roller compacter (Alexander Verlag) to give a granulation product (440.0 g). During the production of formulation 1A, blocking and fluidity failure were not observed.

The granulation processing rate of formulation 1A, comparison formulation 1A and comparison formulation 1B are shown in Table 5. In comparison formulation 1A, the mixture did not proceed to the granulation step due to the fluidity failure, and therefore, the mixture was forcibly pushed into a roller compacter (Alexander Verlag) to give a granulation product.

The formulation of Table 6 shows the amount of each component contained in one uncoated tablet when each granulation product obtained in the above was sieved, the sieved powder (374.72 g), crystalline cellulose (40 g), hydroxypropylcellulose (20 g) and magnesium stearate (5.28 g) were mixed, and tableted with a 8×4.5 mm punch to give a tablet weighing 110 mg.

**[Table 5]**

| | formulation 1A | comparison formulation 1A | comparison formulation 1B |
|---|---|---|---|
| granulation processing rate (g/min) | 458 | 306 | 462 |

**[Table 6]**

| components | formulation 1A | comparison formulation 1A | comparison formulation 1B |
|---|---|---|---|
| | mg/tablet | mg/tablet | mg/tablet |
| (granule) | | | |
| succinate of compound (A) | 66.5 | 66.5 | 40 |
| crystalline cellulose (PH-302) | 23.35 | 23.85 | 50.35 |
| croscarmellose sodium | 3 | 3 | 3 |
| magnesium stearate | 0.33 | 0.33 | 0.33 |
| light anhydrous silicic acid | 0.5 | | - |
| (tableting aid) | | | |
| crystalline cellulose (KG-802) | 10 | 10 | 10 |
| hydroxypropylcellulose | 5 | 5 | 5 |
| magnesium stearate | 1.32 | 1.32 | 1.32 |
| total weight | 110 | 110 | 110 |

As is clear from Table 5, comparison formulation 1A showed a decreased granulation processing rate, and formulation 1A, and comparison formulation 1B did not show a decreased granulation processing rate but showed a moderate granulation processing rate.

From the above-mentioned results, it has been clarified that blocking and fluidity failure during granulation occur when the mixture to be granulated has a high compound (A) content, and the occurrence of such inconveniences can be prevented by the addition of a fluidizer. That is, the tablet of the present invention was shown to inhibit the occurrence of blocking and fluidity failure and has superior productivity.

### Experimental Example 2

In formulation 2A, according to the formulation of Table 7, succinate (monosuccinate) of compound (A), crystalline cellulose (PH-302), croscarmellose sodium, magnesium stearate and light anhydrous silicic acid were uniformly mixed in a vertical granulator (Powrex Corporation) and processed by a roller compacter (Alexander Verlag) to give a granulation product. The obtained granulation product was sieved by a granulator (power mill P-3, Showa Kako Corporation) to give a sieved powder. To the sieved powder were added crystalline cellulose (KG-802), hydroxypropylcellulose and magnesium stearate, and they were mixed in a plastic bag to give granules for tableting. These granules were tableted in an autograph (AG-1, SHIMADZU Corporation) with a 11x6 mm punch to give an uncoated tablet weighing 220 mg. In comparison formulation 2B, according to the formulation of Table 7, an uncoated tablet (220 mg) was obtained in the same manner as in the above-mentioned formulation 2A except that hydroxypropylcellulose was not added. The formulation of the obtained uncoated tablet is shown in Table 7. In addition, the hardness per unit fracture area of the obtained uncoated tablet is shown in Table 8.

| components | formulation 2A | formulation 2B |
|---|---|---|
| | mg/tablet | mg/tablet |
| (granule) | | |
| | | |
| succinate of compound (A) | 133 | 133 |
| crystalline cellulose (PH-302) | 46.7 | 46.7 |
| croscarmellose sodium | 6 | 6 |
| magnesium stearate | 0.66 | 0.66 |
| light anhydrous silicic acid | 1 | 1 |
| (tableting aid) | | |
| | | |
| crystalline cellulose (KG-802) | 20 | 30 |
| hydroxypropylcellulose | 10 | - |
| magnesium stearate | 2.64 | 2.64 |
| total weight | 220 | 220 |

formulation 2B is a comparison formulation

**[Table 8]**

| | formulation 2A | formulation 2B |
|---|---|---|
| punch and die used | 11×6 mm oval | |
| tablet thickness | 4.2 mm | 4.2 mm |
| average hardness (N) (n=5) | 119 | 90.8 |
| fracture area (mm²) | 46.2 | |
| hardness (N/mm²) per unit fracture area | 2.58 | 1.97 |

formulation 2B is a comparison formulation

As shown in Table 8, formulation 2A containing two kinds of binders (crystalline cellulose and hydroxypropylcellulose) has a moderate tablet hardness (not less than 2.1 N/mm²). The above results show that the tablet of the present invention has a moderate tablet hardness when it contains crystalline cellulose and a binder other than crystalline cellulose, prevents breakage of tablet surface and crack of tablet in film coating step, and shows superior productivity.

### Experimental Example 3

The tablets of Example 1 and Example 2 were blister-packed, placed in an aluminum bag, and stored for 6 months under conditions of 40°C and 75%RH. The mass of the remaining compound (A) and total analogs derived from compound (A) was measured by HPLC to evaluate the stability. The results are shown in Table 9.

**[Table 9]**

| | Example 1 | | Example 2 | |
|---|---|---|---|---|
| | start of storage experiment | after storage for 6 months | start of storage experiment | after storage for 6 months |
| content (%) | 99.0 | 100.0 | 99.9 | 100.1 |
| total analogs (%) | 0.44 | 0.41 | 0.44 | 0.41 |

As shown in Table 9, the tablet of the present invention was shown to be superior in the chemical stability.

### Experimental Example 4

The tablets of Example 1 and Example 2 were evaluated for the dissolution property of compound (A) according to the Paddle Method (75 rpm) using 0.01N hydrochloric acid (37°C, 900 mL). The results are shown in Table 10. Each value in the Table shows an average dissolution rate of 6 tablets. The storage conditions were the same as in Experimental Example 3, and the tablets of Example 1 and Example 2 were blister-packed, placed in an aluminum bag, and stored for 6 months under conditions of 40°C and 75%RH.

**[Table 10]**

| | | Example 1 | | Example 2 | |
|---|---|---|---|---|---|
| | | start of storage experiment | after storage for 6 months | start of storage experiment | after storage for 6 months |
| dissolution rate (%) | 0 min | 0 | 0 | 0 | 0 |
| | 10 min | 98 | 97 | 79 | 89 |
| | 15 min | 99 | 99 | 90 | 95 |
| | 20 min | 99 | 99 | 94 | 97 |
| | 30 min | 100 | 99 | 97 | 99 |
| | 45 min | 100 | 99 | 99 | 99 |

As shown in Table 10, the tablet of the present invention was shown to be stable, with no change in the dissolution property of compound (A) before and after the storage.

### Industrial Applicability

The present invention can provide a tablet with a high content of compound (A) or a salt thereof and a size permitting easy administration.

## Claims

1. A tablet comprising 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof, light anhydrous silicic acid, crystalline cellulose and hydroxypropylcellulose, wherein the content of 2-[[6-[(3R)-3-amino-1-piperidinyl]-3,4-dihydro-3-methyl-2,4-dioxo-1(2H)-pyrimidinyl]methyl]-4-fluorobenzonitrile under free form is 35 - 50 weight%.

2. The tablet according to claim 1, wherein the content of crystalline cellulose is 10 - 50 weight%, and the content of hydroxypropylcellulose is 3 - 10 weight%.

3. The tablet according to any one of claims 1 to 2, wherein the content of 2-[[6-[(3R)-3-amino-1-piperidinyl]-3,4-dihydro-3-methyl-2,4-dioxo-1(2H)-pyrimidinyl]methyl]-4-fluorobenzonitrile under free form is more than 40 weight% to not more than 50 weight%.

4. The tablet according to any one of claims 1 to 2, having a dosage form of once per week.

5. A method of producing a tablet comprising 35 - 50 weight% of 2-[[6-[(3R)-3-amino-1-piperidinyl]-3,4-dihydro-3-methyl-2,4-dioxo-1(2H)-pyrimidinyl]methyl]-4-fluorobenzonitrile under free form, comprising a step of granulating a mixture comprising 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof and light anhydrous silicic acid, and a step of tableting the granulation product obtained by the above step,
wherein the method further comprises a step of mixing, after the granulation step, the granulation product obtained by the granulation step, crystalline cellulose and hydroxypropylcellulose, wherein the granulation product obtained by the mixing step is tableted in the tableting step.

6. A tablet obtained by the production method according to claim 5.

## Patentansprüche

1. Tablette umfassend 2-[[6-[(3R)-3-Amino-1-piperidinyl]-3,4-dihydro-3-methyl-2,4-dioxo-1(2H)-pyrimidinyl]methyl]-4-fluorbenzonitril oder ein Salz davon, leichte wasserfreie Kieselsäure, kristalline Cellulose und Hydroxypropylcellulose, wobei der Gehalt an 2-[[6-[(3R)-3-Amino-1-piperidinyl]-3,4-dihydro-3-methyl-2,4-dioxo-1(2H)-pyrimidinyl]methyl]-4-fluorbenzonitril in freier Form 35 - 50 Gew.-% beträgt.

2. Tablette nach Anspruch 1, wobei der Gehalt an kristalliner Cellulose 10 - 50 Gew.-% beträgt, und der Gehalt an Hydroxypropylcellulose 3 - 10 Gew.-% beträgt.

3. Tablette nach einem der Ansprüche 1 bis 2, wobei der Gehalt an 2-[[6-[(3R)-3-Amino-1-piperidinyl]-3,4-dihydro-3-methyl-2,4-dioxo-1(2H)-pyrimidinyl]methyl]-4-fluorbenzonitril in freier Form mehr als 40 Gew.-% bis nicht mehr als 50 Gew.-% beträgt.

4. Tablette nach einem der Ansprüche 1 bis 2, mit einer Darreichungsform von einmal pro Woche.

5. Verfahren zur Herstellung einer Tablette umfassend 35 - 50 Gew.-% von 2-[[6-[(3R)-3-Amino-1-piperidinyl]-3,4-dihydro-3-methyl-2,4-dioxo-1(2H)-pyrimidinyl]methyl]-4-fluorbenzonitril in freier Form, mit einem Schritt des Granulierens einer Mischung umfassend 2-[[6-[(3R)-3-Amino-1-piperidinyl]-3,4-dihydro-3-methyl-2,4-dioxo-1(2H)-pyrimidinyl]methyl]-4-fluorbenzonitril oder ein Salz davon und leichte wasserfreie Kieselsäure, und einem Schritt des Tablettierens des durch den obigen Schritt erhaltenen Granulierungsprodukts, wobei das Verfahren ferner nach dem Granulierungsschritt einen Schritt des Mischens des durch den Granulierungsschritt erhaltenen Granulierungsprodukts mit kristalliner Cellulose und Hydroxypropylcellulose umfasst, wobei das durch den Schritt des Mischens erhaltene Granulierungsprodukt in dem Tablettierschritt tablettiert wird.

6. Tablette, die man mit dem Herstellungsverfahren nach Anspruch 5 erhält.

## Revendications

1. Comprimé comprenant du 2-{[6-((3R)-3-amino-pipéridin-1-yl)-3,4-dihydro-3-méthyl-2,4-dioxo-pyrimidin-1(2H)-yl]-méthyl}-4-fluoro-benzonitrile ou de l'un de ses sels, de la silice colloïdale anhydre, de la cellulose cristalline et de l'hydroxypropyl-cellulose, dont la teneur en 2-{[6-((3R)-3-amino-pipéridin-1-yl)-3,4-dihydro-3-méthyl-2,4-dioxo-pyrimidin-1(2H)-yl]-méthyl}-4-fluoro-benzonitrile à l'état libre vaut de 35 à 50 % en poids.

2. Comprimé conforme à la revendication 1, contenant de 10 à 50 % en poids de cellulose cristalline et de 3 à 10 % en poids d'hydroxy-propyl-cellulose.

3. Comprimé conforme à l'une des revendications 1 et 2,
qui contient de plus de 40 % en poids à pas plus de 50 % en poids de 2-{[6-((3R)-3-amino-pipéridin-1-yl)-3,4-dihydro-3-méthyl-2,4-dioxo-pyrimidin-1(2H)-yl]-méthyl}-4-fluoro-benzonitrile à l'état libre.

4. Comprimé conforme à l'une des revendications 1 et 2,
dont la posologie est d'une fois par semaine.

5. Procédé de production d'un comprimé comprenant de 35 à 50 % en poids de 2-{[6-((3R)-3-amino-pipéridin-1-yl)-3,4-dihydro-3-méthyl-2,4-dioxo-pyrimidin-1(2H)-yl]-méthyl}-4-fluoro-benzonitrile à l'état libre, lequel procédé comporte une étape de granulation d'un mélange comprenant du 2-{[6-((3R)-3-amino-pipéridin-1-yl)-3,4-dihydro-3-méthyl-2,4-dioxo-pyrimidin-1(2H)-yl]-méthyl}-4-fluoro-benzonitrile ou de l'un de ses sels et de la silice anhydre colloïdale,
et une étape où l'on fabrique des comprimés du produit de granulation obtenu à l'issue de l'étape ci-dessus,
et lequel procédé comporte en outre une étape consistant à mélanger, après l'étape de granulation, le produit de granulation obtenu à l'issue de l'étape de granulation, de la cellulose cristalline et de l'hydroxy-propyl-cellulose, étant entendu que c'est le produit de granulation obtenu à l'issue de cette étape de mélange dont on fait des comprimés, dans l'étape de fabrication de comprimés.

6. Comprimé obtenu par un procédé de production conforme à la revendication 5.
